# EUROPÄISCHE PATENTSCHRIFT

(11) **EP 0 823 282 B1**
(45) Veröffentlichungstag und Bekanntmachung des Hinweises auf die Patenterteilung: **14.11.2001**
(21) Anmeldenummer: 97107514.8
(22) Anmeldetag: 07.05.1997
(51) Int. Cl.: B01J 31/24, C07C 45/50

(54) **Verfahren zur Herstellung von Aldehyden**
Aldehydes preparation process
Procédé de préparation d'aldéhydes

(30) Priorität: 13.08.1996 DE 19632600
(43) Veröffentlichungstag der Anmeldung: 11.02.1998
(73) Patentinhaber: Celanese Chemicals Europe GmbH, 60439 Frankfurt am Main (DE)
(72) Erfinder: Bahrmann, Helmut, Dr., 46499 Hamminkeln (DE); Müller, Thomas, 46535 Dinslaken (DE); Lukas, Rainer, Dr., 45133 Essen (DE)

(56) Entgegenhaltungen:
- EP-A- 0 158 246
- EP-A- 0 216 315
- EP-A- 0 246 475
- EP-A- 0 374 615
- EP-A- 0 810 029
- GB-A- 1 312 076
- US-A- 4 716 250

## Beschreibung

Die vorliegende Erfindung betrifft ein Verfahren zur Herstellung von Aldehyden durch Umsetzung von olefinisch ungesättigten Verbindungen mit Wasserstoff und Kohlenmonoxid in homogener Phase und in Gegenwart eines Rhodium-Komplexverbindungen sowie aromatische Phosphine im molaren Überschuß enthaltenden Katalysatorsystems und Abtrennung des Katalysatorsystems vom Reaktionsprodukt durch Membranfiltration.

Die technisch in großem Umfang durchgeführte Hydroformylierung von Olefinen erfolgt zunehmend in Gegenwart von Katalysatorsystemen auf der Basis von Rhodium-Komplexverbindungen, die tertiäre Phosphine oder Phosphite als Liganden enthalten. Da die Liganden in der Regel im Überschuß vorliegen, besteht das Katalysatorsystem aus metallorganischer Komplexverbindung und überschüssigem freiem Ligand. Entsprechend der Löslichkeit dieser Katalysatorsysteme in organischen Medien erfolgt die Hydroformylierung in homogener Phase. Zur Abtrennung der Reaktionsprodukte und Wiedergewinnung des im Reaktionsprodukt homogen gelösten Katalysatorsystems destilliert man das Reaktionsprodukt im allgemeinen aus dem Reaktionsgemisch ab. Dies ist aber wegen der thermischen Empfindlichkeit der gebildeten Aldehyde nur bei der Hydroformylierung von niederen Olefinen mit bis zu etwa 8 Kohlenstoffatomen im Molekül möglich. Bei der Hydroformylierung langkettiger Olefine oder olefinischer Verbindungen mit funktionellen Gruppen werden thermisch empfindliche Produkte oder Produkte mit hohem Siedepunkt gebildet, die sich destillativ nicht mehr zufriedenstellend vom Katalysator abtrennen lassen: Die thermische Belastung des Destillationsgutes führt durch Dickölbildung zu erheblichen Verlusten an Wertprodukt sowie durch Zersetzung der Komplexverbindungen zu Verlusten an Katalysator. Hierdurch wird die wirtschaftliche Attraktivität des Verfahrens entscheidend verringert.

Um die Abtrennung des Katalysatorsystems auf thermischem Weg zu vermeiden, wurden verschiedene Verfahrensalternativen entwickelt. Aus EP-A-0 216 375 ist ein Verfahren zur Herstellung von Aldehyden durch Umsetzung von Olefinen mit Wasserstoff und Kohlenmonoxid in homogener Phase in Gegenwart eines Rhodium sowie aromatische Phosphine im molaren Überschuß enthaltenden Katalysatorsystems bekannt, bei dem als aromatische Phosphine in organischen Medien lösliche und in Wasser unlösliche Salze sulfonierter oder carboxylierter Triarylphosphine eingesetzt werden.

Bei den Kationen dieser Salze handelt es sich um Ammoniumionen der Formeln (NR₂H₂)⁺ und/oder (NR₃H)⁺, wobei R für Alkylreste mit 4 bis 12 Kohlenstoffatomen oder für Aryl- oder Cycloalkylreste mit 6 bis 12 Kohlenstoffatomen steht.

Zur Abtrennung des Katalysatorsystems vom Reaktionsprodukt behandelt man in diesem Fall das Hydroformylierungsgemisch zunächst mit einer Base, z.B. Alkali- oder Erdalkalihydroxid-Lösungen. Hierbei werden aus den (NR₂H₂)⁺-bzw. (NR₃H)⁺-Salzen die entsprechenden sekundären oder tertiären Amine freigesetzt, und gleichzeitig wird ein wasserlösliches Alkali- oder Erdalkalisalz des sulfonierten oder carboxylierten Triarylphosphins gebildet, welches dadurch in die wäßrige Phase gelangt und über eine Extraktion zusammen mit dem komplex am Phosphor gebundenen Rhodium von der das Hydroformylierungsprodukt enthaltenden organischen Phase abgetrennt werden kann.

Aus EP-A-0 374 615 ist es ferner bekannt, daß sich metallorganische Komplexverbindungen, die Phosphor(III)-verbindungen als Liganden enthalten, nach ihrem Einsatz als Katalysatoren für die Hydroformylierung von Olefinen in homogener Phase durch Membranfiltration von den Hydroformylierungsprodukten abtrennen lassen. Unter Einsatz selektiver semipermeabler Polyaramid-Trennmembranen ist es möglich, die metallorganischen Komplexverbindungen unversehrt, d.h. ohne Abbau der katalytisch aktiven Metallverbindung, abzutrennen und zurückzugewinnen. Als treibende Kraft für den Trennvorgang kann hierbei sowohl eine Druckdifferenz (Druckfiltration) als auch eine Konzentrationsdifferenz (Dialyse) dienen.

Als erfolgreich abzutrennende Rhodiumkomplexverbindungen sind in EP-A- 0 374 615 HRhCO[P(C₆H₅)₃]₃, RhCl[P(C₆H₅)₃]₃ und solche Verbindungen genannt, die als Liganden Alkyl-oder Arylammoniumsalze sulfonierter oder carboxylierter Triarylphosphine der allgemeinen Formel 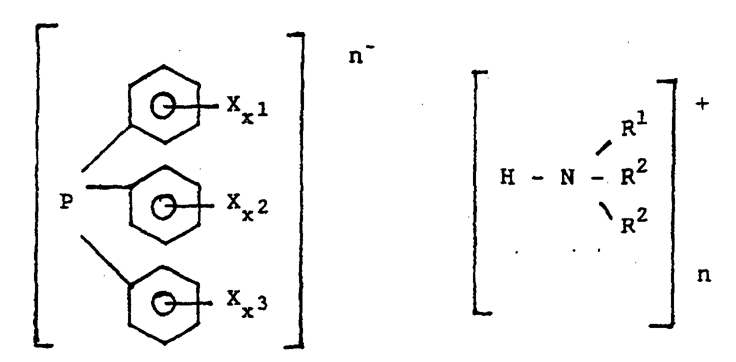 enthalten, wobei X einen Sulfonatrest(SO₃⁻) oder Carboxylatrest(COO⁻) bedeutet, x¹, x² und x³ 0 oder 1 sind, R¹ und R² jeweils gleiche oder verschiedene C₄ - C₁₂-Alkylreste, C₆ - C₁₂-Arylreste oder C6 - C₁₂-Cycloalkylreste bedeuten und R¹ zusätzlich auch für Wasserstoff stehen kann.

Bei der zweistufigen Membranabtrennung eines Rhodium und das Triisooctylammoniumsalz von Tris(m-sulfophenyl)-phosphin enthaltenden Katalysators vom Rohprodukt der Dicyclopentadien-Hydroformylierung werden gemäß EP-A-0 374 615 99,5 % des Rhodiums und 94,4 % der Phosphor(III)verbindung zurückgehalten. 5,6 % der Phosphor(III)verbindung verbleiben somit im organischen Hydroformylierungsprodukt und können hieraus nur durch aufwendige Maßnahmen, wie eine komplizierte Destillation unter größeren Produktverlusten entfernt werden. Die Flußleistung im stationären Endzustand der Membranfiltration liegt lediglich bei 5 bzw. 10 l/m² h in der ersten bzw. zweiten Membranfiltrationsstufe.

Die europäische Offenlegungsschrift EP-810 029 beschreibt ein durch Rhodium katalysiertes, ligandenunterstütztes Verfahren zur Herstellung von Aldehyden aus olefinisch ungesättigten Verbindungen mit Wasserstoff und Kohlenmonoxid in homogener Phase und die Separation der gebildeten Aldehyde von der Katalysatorlösung mittels Druckfiltration durch eine semipermeable Membran aus einem aromatischen Polyamid.

Kennzeichnend für das nach EP-810 029 bekannte Verfahren ist das Verhältnis der Molmassen des im Überschuß verwendeten Liganden zum erhaltenen Aldehyd, das im allgmeinen zwischen 9 und 30, bevorzugt zwischen 10 und 25, insbesondere zwischen 10 und 15, eingestellt wird, um die Trennwirkung der Membran zu optimieren.

Es bestand daher die Aufgabe, ein Verfahren zur Hydroformylierung von olefinisch ungesättigten Verbindungen in homogener Phase zur Verfügung zu stellen, das hohe Aktivitäts- und Selektivitätswerte liefert und gleichzeitig eine verbesserte Abtrennung des gesamten Katalysatorsystems ermöglicht.

Gelöst wird diese Aufgabe durch ein Verfahren zur Herstellung von Aldehyden durch Hydroformylierung von olefinisch ungesättigten Verbindungen mit Wasserstoff und Kohlenmonoxid in homogener Phase in Gegenwart eines Rhodium-Komplexverbindungen sowie aromatische Phosphine im molaren Überschuß enthaltenden Katalysatorsystems und Abtrennung des Katalysatorsystems vom Reaktionsgemisch der Hydroformylierung durch Druckfiltration an einer semipermeablen Membran aus einem aromatischen Polyamid, dadurch gekennzeichnet, daß die Hydroformylierung bei einem pH-Wert von 2,5 bis 4,3 unter Verwendung eines molaren Phosphin : Rhodium-Verhältnisses von mindestens 60 und bei einer Rhodium-Konzentration von mindestens 10 Gew.-ppm, bezogen auf die eingesetzte olefinisch ungesättigte Verbindung, durchgeführt wird und als aromatische Phosphine Alkyl- und/oder Arylammoniumsalze sulfonierter oder carboxylierter Triarylphosphine der allgemeinen Formel I 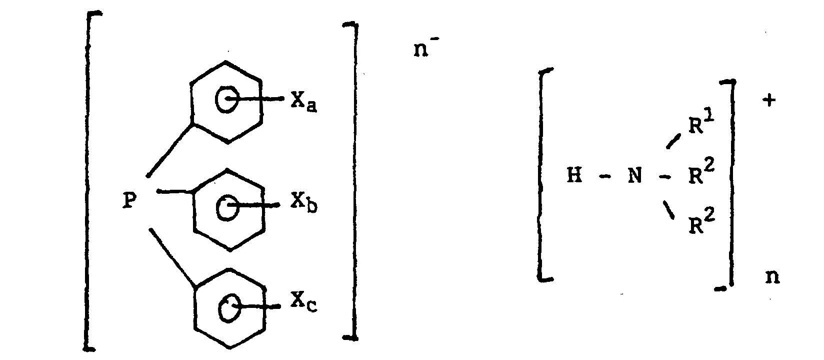 eingesetzt werden, worin X einen Sulfonat-(SO₃⁻)- oder Carboxylat-(COO⁻)-Rest bedeutet, a, b und c gleich oder verschieden und 0 oder 1 sind, wobei mindestens einer der Parameter a, b oder c gleich 1 sein muß, n gleich 1, 2 oder 3 ist, R¹ und R² gleich oder verschieden und C₈ - C₃₀-Alkylreste oder C6 - C₁₀-Aryl- oder -Cycloalkylreste bedeuten und R¹ auch Wasserstoff sein kann und die Summe der Kohlenstoffatome in den Resten R¹ und R² mindestens 30 betragen muß.

Die aromatischen Phosphine der allgemeinen Formel I liegen als Ammoniumcarboxylate oder -sulfonate mit 1 bis 3-fach geladenem Phosphin-Anion und der korrespondierenden Anzahl Ammonium-Kationen als Gegenionen vor. Sie sind in Wasser nicht oder nur in äußerst geringem Maß löslich. In organischen Lösungsmitteln besitzen sie dagegen eine gute bis sehr gute Löslichkeit und eignen sich daher besonders für den Einsatz in organischer Phase.

In der Formel I bedeutet X einen Carboxylat- oder Sulfonatrest, bevorzugt einen Sulfonatrest. a, b und c sind gleich oder verschieden und 0 oder 1, wobei mindestens einer der Parameter a, b oder c gleich 1 sein muß. Bevorzugt sind a, b und c gleich 1.

R¹ und R² sind gleich oder verschieden und bedeuten C₈ - C₃₀-, bevorzugt C₁₂ - C₂₂-Alkylreste, oder C₆ - C₁₀-Aryl-oder -Cycloalkylreste, bevorzugt Phenyl oder Cyclohexylreste, wobei R¹ auch Wasserstoff sein kann.

Somit leiten sich die Ammonium-Kationen [H-NR¹R²R²]⁺ von sekundären oder tertiären Aminen ab und enthalten in den Resten R¹ und R² mindestens 30 und maximal 90, bevorzugt 32 - 70, und besonders bevorzugt 36 - 54 Kohlenstoffatome. Bevorzugt handelt es sich bei den Ammonium-Kationen um das Distearylammoniumion, das Tricetylammoniumion oder das Tri-n-octadecylammoniumion.

Zur Herstellung der Ammoniumsalze des sulfonierten Triphenylphosphins wird zunächst Triphenylphosphin gemäß DE-26 27 354 durch Umsetzung mit überschüssigem Schwefeltrioxid in Form von Oleum sulfoniert, das Sulfonierungsgemisch mit Wasser verdünnt und anschließend das in einem wasserunlöslichen organischen Lösungsmittel gelöste, wasserunlösliche Amin NR¹R²R² zugegeben. Hierbei bilden sich die entsprechenden Ammoniumsalze des sulfonierten Triphenylphosphins, welche als organische Phase abgetrennt werden können.

Die Bildung des Katalysatorsystems aus Rhodium oder einer Rhodium-Verbindung und der Phosphin-Verbindung der Formel I erfolgt entweder in einem der Hydroformylierung vorgeschalteten Schritt, der sogenannten Präformierung, oder aber, insbesondere bei kontinuierlicher Arbeitsweise, in-situ während der Hydroformylierungsreaktion.

Die der Hydroformylierung vorgeschaltete Präformierung erfolgt bevorzugt bereits in dem Reaktor, in dem nachfolgend auch die Hydroformylierung stattfindet, sie kann jedoch auch in einem separaten Reaktionsbehälter durchgeführt werden.

Zur Herstellung des Katalysatorsystems durch Präformierung wird die Rhodium-Komponente (Rhodium oder eine Rhodiumverbindung) mit der Phosphin-Verbindung gemäß Formel I entweder im Hydroformylierungsreaktor oder in der separaten Vorrichtung zusammengebracht. Hierbei setzt man sowohl das Rhodium oder die Rhodiumverbindung als auch die Phosphin-Verbindung gemäß Formel I in einem organischen Lösungsmittel gelöst oder, im Falle von elementarem Rhodium, suspendiert ein. Geeignete Lösungsmittel sind hierbei organische Lösungsmittel, die unter den Bedingungen der nachfolgenden Hydroformylierung inert sind, wie Toluol, ortho-Xylol, meta-Xylol, para-Xylol, Gemische isomerer Xylole, 2-Ethylhexanol, Ethylbenzol, Mesitylen, Gemische dieser Verbindungen oder aliphatische Kohlenwasserstoffe. Bevorzugt wird o-Xylol oder Toluol eingesetzt.

Das Rhodium/Phosphin-Gemisch wird anschließend mit einem Gemisch aus Kohlenmonoxid und Wasserstoff beaufschlagt und unter einem Kohlenmonoxid/Wasserstoff-Druck von 0,5 - 27 MPa bei Temperaturen von 80 - 150°C mindestens 1 h umgesetzt, unter Bildung von wasserunlöslichen und in organischen Medien löslichen Rhodium-Komplexverbindungen, die das Phosphin als Liganden enthalten. Sie ergeben zusammen mit dem im organischen Lösungsmittel gelösten Phosphin-Überschuß das Katalysatorsystem. Die Lösung des Katalysatorsystems kann dann, sofern die Herstellung in einer separaten Vorrichtung erfolgt, in den Hydroformylierungsreaktor überführt werden und mit dem zu hydroformylierenden Olefin versetzt werden.

Soll die Herstellung des Katalysatorsystems in-situ während der Hydroformylierungsreaktion erfolgen, so werden die oben beschriebenen Komponenten, Rhodium bzw. Rhodium-verbindung und Phosphin, gleichzeitig mit dem Olefin in den Hydroformylierungsreaktor eingebracht.

Rhodium gelangt entweder als Metall oder als Verbindung zum Einsatz. In metallischer Form verwendet man es in Form feinverteilter Partikel oder in dünner Schicht auf einem Träger wie Aktivkohle, Calciumcarbonat, Aluminiumsilikat oder Tonerde niedergeschlagen. Als Rhodiumverbindungen kommen solche Substanzen in Betracht, die in organischen Lösungsmitteln löslich oder suspendierbar sind oder unter den Reaktionsbedingungen darin löslich oder suspendierbar werden. Geeignet sind die verschiedenen Rhodiumoxide, Salze anorganischer Wasserstoff- oder Sauerstoffsäuren, sowie Salze aliphatischer Mono- oder Polycarbonsäuren. Beispiele für Rhodiumsalze sind Rhodiumnitrat, Rhodiumsulfat, Rhodiumacetat, Rhodium-2-ethylhexanoat, Rhodiummalonat. Rhodiumhalogenverbindungen sind wegen der geringeren Aktivität der resultierenden Komplexe und wegen des korrosiven Verhaltens der Halogenidionen dagegen weniger geeignet. Weiterhin können Rhodiumcarbonylverbindungen wie Rh₄(CO)₁₂ oder Rh₆(CO)₁₆ oder Komplexsalze des Rhodiums, z.B. Cyclooctadienylrhodiumverbindungen eingesetzt werden. Bevorzugt ist Rhodiumoxid, besonders bevorzugt sind Rhodiumacetat und Rhodium-2-ethylhexanoat.

Es ist nicht erforderlich, die Phosphin-Liganden der Formel I im Katalysatorsystem als einheitliche Verbindungen zu verwenden. Es können z.B. auch unterschiedliche Sulfonierungsstufen der Phosphine und/oder Sulfonatgemische mit verschiedenen Ammonium-Kationen eingesetzt werden.

Es hat sich bei der Bildung und der Verwendung des Katalysatorsystems bewährt, Rhodium und die aromatischen Phosphine der Formel I nicht in stöchiometrischem Verhältnis, also entsprechend der chemischen Zusammensetzung der sich bildenden Rhodium-Komplexverbindung zu verwenden, sondern die aromatischen Phosphine im Überschuß einzusetzen. Dabei ist es wesentlich, daß je mol Rhodium mindestens 60 mol Phosphin eingesetzt werden. Bevorzugt wird ein molares Verhältnis von Rhodium zu aromatischem Phosphin von 1 : (60 - 120). Besonders bevorzugt ist ein Verhältnis von 1 : (70 - 110), und insbesondere wird ein Verhältnis von 1 : (80 - 100) verwendet.

Es hat sich außerdem als wesentlich herausgestellt, daß die Rhodium-Konzentration in der Hydroformylierung, bezogen auf die olefinisch ungesättigte Verbindung mindestens 10 Gew.-ppm, bevorzugt mindestens 20 Gew.-ppm und besonders bevorzugt 60 bis 150 Gew.-ppm beträgt.

Im erfindungsgemäßen Verfahren werden olefinisch ungesättigte Verbindungen mit 2 bis 30 Kohlenstoffatomen umgesetzt, die eine oder mehrere Doppelbindungen besitzen können. Geeignet sind substituierte oder unsubstituierte Alkene mit 6 bis 30 Kohlenstoffatomen, substituierte oder unsubstituierte Diene mit 4 bis 10 Kohlenstoffatomen, substituierte oder unsubstituierte Cycloalkene oder Dicycloalkene mit 5 bis 12 Kohlenstoffatomen im Ringsystem, Ester einer ungesättigten Carbonsäure mit 3 bis 20 Kohlenstoffatomen und eines aliphatischen Alkohols mit 1 bis 18 Kohlenstoffatomen, Ester einer gesättigten Carbonsäure mit 2 bis 20 Kohlenstoffatomen und eines ungesättigten Alkohols mit 2 bis 18 Kohlenstoffatomen, ungesättigte Alkohole oder Ether mit jeweils 3 bis 20 Kohlenstoffatomen oder araliphatische Olefine mit 8 bis 20 Kohlenstoffatomen.

Bei den substituierten oder unsubstituierten Alkenen mit 6 bis 30 Kohlenstoffatomen kann es sich um geradkettige oder verzweigte Alkene mit endständiger oder innenständiger Lage der Doppelbindung handeln. Bevorzugt sind geradkettige Olefine mit 6 bis 18 Kohlenstoffatomen wie n-Hexen-1, n-Hepten-1, n-Octen-1, n-Nonen-1, n-Decen-1, n-Undecen-1, n-Dodecen-1, n-Octadecen-1 und acyclische Terpene. Geeignet sind auch verzweigte Alkene wie Diisobutylen (2,4,4-Trimethylpenten-1), Tripropylen, Tetrapropylen und Dimersol (Dibutylen).

Bevorzugte Beispiele für unsubstituierte Diene mit 4 bis 10 Kohlenstoffatomen sind 1,3-Butadien, 1,5-Hexadien und 1,9-Decadien.

Beispiele für substituierte und unsubstituierte Cycloalkene oder Dicycloalkene mit 5 bis 12 Kohlenstoffatomen im Ringsystem sind Cyclohexen, Cycloocten, Cyclooctadien, Dicyclopentadien und cyclische Terpene wie Limonen, Pinen, Camphoren und Bisabolen. Bevorzugt ist Dicyclopentadien.

Beispielhaft für araliphatische Olefine mit 8 bis 20 Kohlenstoffatomen steht Styrol.

Als Beispiele für Ester einer ungesättigten Carbonsäure mit 3 bis 20 Kohlenstoffatomen und eines aliphatischen Alkohols mit 1 bis 18 Kohlenstoffatomen seien die Acrylsäureester und die Methacrylsäureester mit 1 - 18 Kohlenstoffatomen in der Alkohol-Komponente genannt.

Zu den Estern einer gesättigten Carbonsäure mit 2 - 20 Kohlenstoffatomen und eines ungesättigten Alkohols mit 2 - 18 Kohlenstoffatomen gehören die Vinyl- und Allylester mit 2 - 20 Kohlenstoffatomen in der Carbonsäurekomponente, beispielsweise Vinylacetat.

Zu den ungesättigten Alkoholen und Ethern zählen beispielsweise die Allylalkohole und Vinylether.

Die Umsetzung des Olefins mit Kohlenmonoxid und Wasserstoff erfolgt bei einer Temperatur von 100 bis 140°C, bevorzugt 120 bis 130°C und unter einem Druck von 0,5 bis 27 MPa, bevorzugt 20 bis 25 MPa. Die Zusammensetzung des Synthesegases, d.h. das Volumenverhältnis von Kohlenmonoxid und Wasserstoff kann sich über weite Bereiche erstrecken und z.B. zwischen 1 : 10 bis 10 : 1 variiert werden. Im allgemeinen setzt man Gasgemische ein, in denen das Volumenverhältnis von Kohlenmonoxid und Wasserstoff etwa 1 : 1 ist oder von diesem Wert nur wenig abweicht.

Von großer Bedeutung ist ferner, daß die Hydroformylierung bei einem pH-Wert von 2,5 - 4,3, bevorzugt 3,0 - 4,0, insbesondere 3,5 durchgeführt wird. Im Verlauf der Hydroformylierung kann der pH-Wert kontinuierlich durch Dissoziation des Ammoniumsalzes des sulfonierten oder carboxylierten Triphenylphosphins in freies Amin und die entsprechende Sulfonsäure- bzw. Carbonsäureform des Triphenylphosphins sinken. Zur Einstellung des obengenannten pH-Werts wird gegebenenfalls freies Amin NR¹R²R² oder ein Metallhydroxid in entsprechenden Mengen zugesetzt.

Bevorzugt führt man das erfindungsgemäße Verfahren in Anwesenheit eines organischen Lösungsmittels durch, welches unter den Bedingungen der Hydroformylierung inert ist und zudem die Membran in der Membranfiltrationsstufe nicht angreift. Geeignete Lösungsmittel sind aromatische Kohlenwasserstoffe, z.B. Toluol, ortho-Xylol, meta-Xylol, para-Xylol, Gemische isomerer Xylole, Ethylbenzol, Mesitylen, Gemische dieser Verbindungen oder aliphatische Kohlenwasserstoffe. Aber auch polarere Lösungsmittel, wie Acetophenon, Tetrahydrofuran, Sulfinol, Glykole oder Polyglykole, können verwendet werden. Das erfindungsgemäße Verfahren kann jedoch auch ohne Zusatz eines organischen Lösungsmittels durchgeführt werden, wobei in diesem Fall die olefinische Ausgangsverbindung und das gebildete Hydroformylierungsprodukt als Lösungsmittel wirken. Aufgrund der gewöhnlich höheren Viskosität eines solchen Reaktionsgemisches werden bei der Membranfiltration dann aber nur geringere Flußleistungen erreicht.

Die Herstellung der Aldehyde durch Umsetzung der in flüssiger und gasförmiger Phase vorliegenden Reaktionspartner erfolgt in konventionellen Reaktoren und kann sowohl kontinuierlich als auch diskontinuierlich durchgeführt werden.

Nach Beendigung der Hydroformylierung wird das Reaktionsgemisch in der Regel gekühlt, durch Entspannen von gasförmigen Bestandteilen befreit und mit einem Inertgas wie z.B. Stickstoff oder mit einer Synthesegasmischung aus CO und H₂ überlagert. Anschließend erfolgt die Auftrennung mittels Membranfiltration. Das Reaktionsgemisch kann jedoch auch ohne Kühlung der Membrantrennung zugeführt werden.

In dem zur Membranfiltration eingesetzten Reaktionsgemisch der Hydroformylierung beträgt die Konzentration der im Überschuß vorliegenden aromatischen Phosphine der Formel I 2,5 - 25, bevorzugt 5 - 15 Gew.-%, bezogen auf das gesamte zur Membranfiltration eingesetzte Reaktionsgemisch.

Die Konzentration der Rhodium-Komplexverbindungen in dem zur Membranfiltration eingesetzten Reaktionsgemisch der Hydroformylierung beträgt 2 - 400 Gew.-ppm., bevorzugt 10 - 300 Gew.-ppm, insbesondere 50 - 150 Gew.-ppm, bezogen auf das gesamte zur Membranfiltration eingesetzte Reaktionsgemisch.

Die Membranfiltration erfolgt an einer semipermeablen Membran aus einem aromatischen Polyamid unter einem Druck von 0,1 - 15, bevorzugt 0,5 - 5, insbesondere 1 - 2 MPa.

Die Membranfiltration kann ein- oder mehrstufig durchgeführt werden, bevorzugt erfolgt sie mehrstufig, insbesondere zweistufig. Sie kann entweder mit parallel oder mit in Reihe geschalteten Trennstufen durchgeführt werden.

Bevorzugt wird die Reihenschaltung, bei der in jeder Stufe das Retentat abgetrennt und die Permeat-Lösung der nächstfolgenden Trennstufe zugeleitet wird. Eine derartige Reihenschaltung erlaubt eine besonders effektive Nutzung des vorhandenen Systemdruckes, d.h. des Arbeitsdruckes im vorausgegangenen Verfahrensschritt.

Zu besonders guten Trennleistungen gelangt man, wenn die Gesamtretentatmenge 8 - 90, bevorzugt 10 - 70, besonders bevorzugt 15 - 50 und insbesondere 20 - 40 %, bezogen auf das zur Membranfiltration eingesetzte Reaktionsgemisch, beträgt und die Konzentration der abgetrennten aromatischen Phosphine der allgemeinen Formel I im Retentat der Membranfiltration mindestens dreimal so groß ist, wie in dem zur Membranfiltration eingesetzten Reaktionsgemisch der Hydroformylierung.

Bei der zweistufigen Membranfiltration hat es sich ferner bewährt, daß das Verhältnis der Retentatmenge der 1. Filtrationsstufe zur Retentatmenge der 2. Filtrationsstufe etwa 1 : 1 beträgt.

Eine weitere Erhöhung der Trennleistung der Membran bei Anwendung der vorstehend beschriebenen Verfahrensvarianten erreicht man durch Erhöhung der Überströmung der Membran mit Hilfe einer Umwälzpumpe. Die lineare Strömungsgeschwindigkeit über die Membran liegt üblicherweise im Bereich von 0,1 - 10 m/sec, vorzugsweise 0,5 - 2,5 m/sec.

Die das Katalysatorsystem enthaltenden Retentate der Trennstufen können vereinigt werden und wieder in die Hydroformylierung zurückgeführt werden, gegebenenfalls unter ergänzendem Zusatz des Rhodiums und/oder der Rhodium-Komplexverbindungen sowie der aromatischen Phosphine der allgemeinen Formel I. Der Zusatz dieser ergänzenden Mengen kann bei zweistufiger Durchführung der Membranfiltration auch bereits zum Permeat der 1. Stufe vor dessen Zuleitung zur 2. Membranfiltrationsstufe erfolgen. Auf diese Weise wird ein verbessertes Trennergebnis erreicht und ein mehrfacher Wiedereinsatz des Katalysatorsystems in der Hydroformylierung ermöglicht, ohne daß nennenswerte Einbußen hinsichtlich Aktivität und Selektivität des Katalysatorsystems auftreten.

Wird das erfindungsgemäße Verfahren in Gegenwart eines Lösungsmittels durchgeführt, so kann eine besonders hohe Gesamteffizienz sowohl des Hydroformylierungs- als auch des Membrantrennschrittes erreicht werden, wenn man die Hydroformylierungsstufe mit wenig Lösungsmittel zur Erzielung eines möglichst hohen Umsatzes, die Membranstufe jedoch mit viel Lösungsmittel zur Absenkung der Viskosität betreibt. In der Hydroformylierungsstufe hat sich eine Lösungsmittelkonzentration von 5 - 25 Gew.-%, bevorzugt 7 - 13 Gew.-%, bezogen auf das gesamte Reaktionsgemisch der Hydroformylierung, bewährt, im Membranfiltrationsschritt sind dagegen 30 - 70 Gew.-%, insbesondere 40 - 60 Gew.-% Lösungsmittel, bezogen auf das gesamte zur Membranfiltration eingesetzte Reaktionsgemisch, bevorzugt. Diese höhere Lösungsmittelkonzentration in der zur Membranfiltration eingesetzten Reaktionsmischung erreicht man, indem man aus den vereinigten Permeaten der Membranfiltrationstrennstufen das organische Lösungsmittel destillativ abtrennt und vor die Membranfiltration zurückführt. Dort wird es wieder dem aufzutrennenden Reaktionsgemisch der Hydroformylierung zugesetzt. Hierdurch wird die entsprechende Verdünnung erreicht, die zur Erzielung hoher Flußleistungen dient.

Die erfindungsgemäß eingesetzten Membranen bestehen aus einem aromatischen Polyamid, auch Polyaramid genannt. Man erhält die Polyaramide durch Polykondensation aus aromatischen Dicarbonsäuren bzw. Dicarbonsäurederivaten und aromatischen Diaminen in einem dipolar aprotischen Lösungsmittel. Als Carbonsäurekomponente kommen z.B. Terephthalsäure, 4,4'-Diphenyldicarbonsäure, 4,4'-Diphenyletherdicarbonsäure, 4,4'-Diphenylsulfondicarbonsäure oder 2,6-Napthalindicarbonsäure in Betracht. Als Diaminkomponente sind p-Phenylendiamin, 3,3'-Dimethoxybenzidin, 3,3'-Dichlorbenzidin, 3,3'-Dimethylbenzidin, 4,4'-Diaminodiphenylmethan, 2,2-Bis-(4-aminophenyl)propan oder 1,4-Bis(4-aminophenoxy)benzol geeignet.

Besondere Bedeutung haben Membranen aus solchen Polyaramiden, die neben einer Carbonsäurekomponente verschiedene Diamine als Monomere enthalten. So haben sich z.B. Polyaramide bewährt, die aus Terephthalsäure, p-Phenylendiamin, 1,4-Bis(4-aminophenoxy)benzol und 3,3'-Dimethylbenzidin aufgebaut sind. In den Polymeren können die Amine statistisch verteilt sein. Die Polyamide können aber auch die Struktur von Blockcopolymerisaten besitzen.

Das mittlere Molekulargewicht der Polyaramide kann sich über einen weiten Bereich erstrecken. Üblicherweise beträgt es 5.000 bis 200.000. Bevorzugt werden Polyaramide mit einer Molmasse von 10.000 bis 50.000.

Zur Herstellung der erfindungsgemäßen Membranen hat sich ein Verfahren bewährt, das in der deutschen Patentanmeldung P 38 02 030 beschrieben ist. Die hier offenbarten Membranen bestehen aus einem Copolyamid, das aus drei unterschiedlichen Diaminen und einer Dicarbonsäure aufgebaut ist. Eine Lösung dieses Copolyamids in einem aprotischen, polaren Lösungsmittel vom Amidtyp, z.B. N-Methyl-2-pyrrolidon, wird auf eine plane Unterlage als flüssige Schicht ausgebreitet. Diese flüssige Schicht trägt man in die Fällflüssigkeit, insbesondere Wasser, ein, die mit dem Lösungsmittel der Lösung mischbar ist, das Polymerisat jedoch als Membran abscheidet. Man läßt die Fällflüssigkeit solange auf die ausgefällte Membran einwirken, bis das Lösungsmittel vollständig durch die Fällflüssigkeit ersetzt ist. Sofern erforderlich, kann die Membran noch einer Wärmebehandlung unterzogen werden. Anschließend wird die Membran, gegebenenfalls nach vorheriger Behandlung mit Glycerin, getrocknet.

Die nach dem vorstehend beschriebenen Verfahren hergestellten Membranen sind integral asymmetrisch und sind dem Fachmann prinzipiell bekannt. Die Membranen besitzen eine sehr dünne, trennaktive Schicht, deren Dicke 0,05 bis 5 µ beträgt und eine poröse Stützstruktur. Die Dicke der aus trennaktiver Schicht und Stützstruktur bestehenden Membran kann 10 bis 400 µ betragen, sie liegt vorzugsweise im Bereich von 50 bis 200 µ

Die Form der Membran kann beliebig gewählt werden. Sie kann als Scheibe und insbesondere als Hohlfaser oder Kapillare ausgebildet sein, aber auch jede andere, für die vorgesehene Verwendung geeignete Gestalt haben. Maßgebend ist die Erzielung einer möglichst hohen Stabilität und überdies einer größtmöglichen Oberfläche je Volumeneinheit, um einen zufriedenstellenden Durchsatz zu erreichen.

Es empfiehlt sich, die Membran vor dem Einsatz vorzubehandeln. Im einfachsten Fall taucht man sie in die zu trennende Lösung. Aber auch andere Konditionierungsverfahren sind möglich. Die zu Lagerungszwecken mit Glycerin getränkte Membran wird zunächst mit Wasser gewaschen und anschließend für 10 Minuten in 80 - 100° heißem Wasser belassen. Anschließend ersetzt man das Wasser z.B. durch i-Propanol, indem man die Membran in i-Propanol einlegt und den Alkohol mehrfach erneuert. Danach wird das i-Propanol in gleicher Weise durch das Reaktionsgemisch der Hydroformylierung ersetzt, in dem die abzutrennenden Rhodium-Komplexverbindungen sowie die aromatischen Phosphine der Formel I gelöst sind. Zur Erzielung einer optimalen Trennleistung hat es sich ferner bewährt, die Membran unter Arbeitsbedingungen eine gewisse Zeit einlaufen zu lassen, d.h. die Membranfiltration unter Verwendung des Reaktionsgemisches der Hydroformylierung durchzuführen, die erhaltenen Retentate und Permeate jedoch wieder zu vereinigen und in das Reaktionsgemisch der Hydroformylierung vor der Membranfiltration zurückzuführen. Durch diese sogenannte Druckkonditionierung schließen sich weitere Poren der Membran, wodurch die Trennleistung der Membran steigt. Art und Methode der Konditionierung der Membran bestimmen die beim erfindungsgemäßen Verfahren einzuhaltenden Arbeitsbedingungen.

Das erfindungsgemäße Verfahren zeichnet sich durch die Kombination von verschiedenen speziellen Hydroformylierungsbedingungen sowie die Verwendung von aromatischen Phosphin-Liganden mit besonderen Ammoniumkationen aus. Werden die zuvor genannten Werte bzw. Wertebereiche für die Rhodium-Konzentration, bezogen auf das eingesetzte Olefin, das Phosphin : Rhodium-Verhältnis und den pH-Wert eingehalten, sowie Phosphin-Liganden gemäß der allgemeinen Formel I eingesetzt, so werden sowohl im Hydroformylierungsschritt ausgezeichnete Selektivitäts- und Aktivitätswerte erhalten als auch bei der nachfolgenden Membranfiltration ausgezeichnete Rückhaltewerte. Bei einer einstufigen Membranfiltration werden 95 - 98 % des Rhodiums sowie mindestens 90 % des Liganden zurückgehalten. Bei zweistufiger Durchführung werden 99 - 99,5 % des Rhodiums und mindestens 97 %, in vielen Fällen sogar mehr als 98 % des Liganden zurückgewonnen. Hierbei ist die gleichzeitige Einhaltung der genannten Wertebereiche für die Parameter als auch die Wahl des richtigen Phosphin-Liganden von Bedeutung. Fällt nur einer der Parameter oder der Ligand aus den genannten Wertebereichen bzw. der allgemeinen Formel I heraus, so hat dies zur Folge, daß entweder bei der Hydroformylierung oder bei der Membranfiltration bzw. in beiden Schnitten negative Auswirkungen zu beobachten sind. Leiten sich die Ammoniumsalze z.B. von tertiären Aminen mit zu hoher Kohlenstoffatom-Zahl in den Resten R¹ und R² ab, so werden aufgrund des hohen sterischen Anspruchs des Amins zwar ausgezeichnete Rückhalteraten für Ligand und Amin erzielt, infolge des hohen Molekulargewichts des Amins führt jedoch die Einstellung des für gute Hydroformylierungsergebnisse notwendigen P:Rh-Verhältnisses von mindestens 60 zum Vorhandensein einer extrem großen Menge an Ligand im Reaktor. Dadurch verkleinert sich das für die übrigen Reaktanden, insbesondere das Olefin, zur Verfügung stehende Reaktorvolumen entsprechend. Dies ist ein Effekt, der zu geringeren Produktdurchsätzen im Hydroformylierungsschritt und damit zu erhöhten Verfahrenskosten führt, was eine geringere wirtschaftliche Attraktivität des Verfahrens nach sich zieht. Wird dagegen ein Amin mit zu geringer Kohlenstoffatom-Anzahl verwendet, z. B. Triisooctylamin, so werden zwar bei der Hydroformylierung ausgezeichnete Selektivitäten erzielt, die Rückhalteraten bei der Membranfiltration sind jedoch deutlich geringer. Wird der angegebene pH-Wert Bereich nicht eingehalten, so kommt es ebenfalls zu einer Beeinträchtigung der Hydroformylierungsreaktion in Form einer geringeren Selektivität. Wird das erfindungsgemäße Verfahren in Gegenwart eines Lösungsmittels durchgeführt, so ist es wesentlich, in der Membranfiltration ein Reaktionsgemisch mit einer Lösungsmittelkonzentration von 30 - 70 Gew.-% einzusetzen. Wird weniger Lösungsmittel verwendet, so sinken die Flußleistungen, so daß zur Erzielung ausreichender Durchsätze entsprechend größere Membranflächen eingesetzt werden müssen. Wird die gleiche Lösungsmittelkonzentration von 30 - 70 Gew.-% aber auch in der Hydroformylierungsstufe verwendet, so sinken der Umsatz und die Produktivität des Verfahrens. Hier ist es daher von Vorteil, nur eine Lösungsmittelkonzentration von 5 - 25 Gew.-% im Reaktionsgemisch zu halten.

### Beispiele

Nachfolgend wird zunächst die Herstellung einer Membran der Art beschrieben, wie sie nach dem erfindungsgemäßen Verfahren eingesetzt werden kann.

### Herstellung der Membran

Das Polyaramid wird durch Kondensation von

| | |
|---|---|
| 97-99 mol% | Terephthalsäuredichlorid |
| 25 mol% | p-Phenylendiamin |
| 25 mol% | 1,4-Bis(4-aminophenoxy)benzol |
| 50 mol% | 3,3'-Dimethylbenzidin |

in N-Methylpyrrolidon als Lösungsmittel hergestellt. Terephthalsäuredichlorid setzt man in einer solchen Menge ein, daß das Polyaramid einen Staudinger-Index von 200 bis 300 ml/g hat. Die Lösungsmittelmenge wird so bemessen, daß eine etwa 7 Gew.-% Polykondensat enthaltende Lösung entsteht. Nachdem die Kondensation erfolgt ist, wird der locker an das Lösungsmittel gebundene Chlorwasserstoff durch Zugabe von 100 mol% CaO neutralisiert. Anschließend löst man in dem Reaktionsgemisch unter Rühren 5 Gew.-% (bezogen auf die Polymerlösung) wasserfreies Calciumchlorid. Die Lösung wird gelinde erwärmt, filtriert und entgast. Sie kann unmittelbar zur Membranherstellung verwendet werden.

Es ist möglich, die Membran trägerfrei oder auf einem Polyestervlies als Träger herzustellen. Im folgenden wird die Herstellung einer trägerfreien Membran beschrieben. Die leicht erwärmte Polyaramidlösung wird mit einer Rakel auf einer Glasplatte zu einem gleichmäßigen Film von etwa 150 µ ausgezogen und in ein Wasserbad von 2°C eingetaucht. Nach etwa 20 min zieht man die Membran von der Glasplatte ab und legt sie 5 min in 100°C heißes Wasser.

Darauf gibt man die Membran in i-Propanol, um die Porenflüssigkeit Wasser gegen den Alkohol auszutauschen. Anschließend wird die Membran mit Toluol gewaschen, nach dieser Behandlung ist sie zur Durchführung der Trennungen geeignet. Bei allen Operationen ist darauf zu achten, daß die Membran nicht austrocknet.

### Beispiele 1 - 5, Vergleichsversuche 1 - 3

Hydroformylierung von Dicyclopentadien (DCP) unter Verwendung von Katalysatorsystemen, die Rhodium und verschiedene Ammoniumsalze des Triphenylphosphintrisulfonats (TPPTS) enthalten:

### a) Herstellung des Distearylammoniumsalzes des TPPTS

253 g einer Na-TPPTS-Lösung werden unter Stickstoff in einem Rührkolben vorgelegt und auf 65°C erwärmt. Anschließend fügt man eine Lösung von 250,3 g Distearylamin in 595 g Toluol hinzu. Innerhalb von 60 min gibt man unter Rühren 90 ml 20%ige Schwefelsäure zu, bis ein pH-Wert von 2,6 erreicht ist, und läßt 2,5 h nachreagieren. Zur besseren Phasentrennung werden 170 g Isopropanol zugegeben. Nach 15 min werden 1037,5 g einer organischen Phase, die das Distearylammoniumsalz des TPPTS mit 0,33 mol TPPTS pro mol Amin enthält, abgetrennt. Andere Ammoniumsalze des TPPTS (Beispiele 2 - 5 und Vergleichsversuche 1 - 3) werden analog zu obiger Vorschrift dargestellt.

### b) Diskontinuierliche Hydroformylierung von Dicyclopentadien

Ein 2,15 1 Rührautoklav wird mit Stickstoff gespült. In einer Glasvorlage mit Stickstoffüberlagerung werden 212,8 g der jeweiligen Ligandlösung aus a) sowie 0,29 mmol Rhodium in Form eines 2-Ethylhexanoatsalzes gelöst (60 Gew.-ppm Rh; P/Rh-Verhältnis: 100) und 500 g Toluol unter Stickstoff in den Autoklaven überführt. Danach wird unter Rühren durch Zufuhr von Synthesegas ein Druck von 27 MPa eingestellt. Nach Erreichen einer Reaktionstemperatur von 130°C läßt man zwei Stunden präformieren. Danach werden innerhalb von 1 Stunde 500 g Dicyclopentadien in den Autoklaven gepumpt. Durch Kühlung mit einem Luftgebläse wird die Temperatur von 130°C gehalten. Nach Ende der Dicyclopentadien-Zufuhr läßt man noch 3 Stunden nachreagieren. Der pH-Wert liegt dabei im Bereich zwischen 3,5 und 4,3. Anschließend wird der Autoklav auf Raumtemperatur abgekühlt und entspannt. Der Autoklaveninhalt wird dann mit Restdruck in einen 2 1 Dreihalskolben mit Tauchstutzen überführt und gewogen. Aus der Gewichtszunahme errechnet sich jeweils der in Tabelle 1 angegebene Dicyclopentadienumsatz.

Die Hydroformylierung des Dicyclopentadiens unter Verwendung der Ammoniumsalze des TPPTS gemäß den Beispielen 2 - 5 und der Vergleichsversuche 1 - 3 wird analog durchgeführt. Die erhaltenen Ergebnisse sind in Tabelle 1 zusammengefaßt.

### c) Einstufige Membranfiltration

Das jeweilige obige Reaktionsprodukt aus b) wird über eine Labormembranfilteranlage gegeben. Als Membran wird eine Polyaramidmembran der Firma Hoechst AG (UF-PA (PET 100)) verwendet. Die Membran wird zunächst 10 min bei 80°C in Wasser getempert. Mittels einer Umwälzpumpe wird die Membran dann mit 200 l/h überströmt und ein Druck von 1 MPa eingestellt. Bei einer Betriebstemperatur von 40°C passiert die in Tabelle 1 angegebene Menge des Hydroformylierungsproduktes die Membran als Permeat. Im Permeat wird der Gehalt an Katalysatorbestandteilen bestimmt, woraus sich, bezogen auf das eingesetzte Reaktionsgemisch der Hydroformylierung, die in Tabelle 1 angegebenen Rückhaltewerte ergeben.

### Beispiele 6 und 7, Vergleichsversuch 4

Kontinuierliche Hydroformylierung von Dicyclopentadien (Beispiele 6 und 7: Verwendung des Distearylammoniumsalzes des TPPTS als Ligand im Rhodium-Katalysatorsystem; Vergleichsversuch 4: Verwendung des Methyldistearylammoniumsalzes des TPPTS als Ligand im Rhodium-Katalysatorsystem)

Die Hydroformylierung wird kontinuierlich in einem 17 1 Druckrohr unter den in Tabelle 2 angegebenen Reaktionsbedingungen durchgeführt.

Bei Einsatz des Methyldistearylammoniumsalzes des TPPTS (Vergleichsversuch 4) wird der Einfluß eines niedrigen P/Rh-Verhältnisses von 15 deutlich. Es zeigt sich, daß ein Rhodium-Verlust von 15 % im Verlauf der Hydroformylierung eintritt. Beim Übergang zu einem höheren P/Rh-Verhältnis werden keine Rh-Verluste in der Hydroformylierung mehr beobachtet (Beispiel 6).

Das kontinuierlich anfallende Produkt aus Beispiel 7 wird nach der Hydroformylierung auf 40°C gekühlt, in mit Stickstoff überlagerten Wechselvorlagen zwischengelagert und anschließend der Membranfiltration unterworfen. Um eine hohe Retention von Ligand und Rh-Komplexverbindung zu erhalten, wird die Membranfiltration dabei zweistufig durchgeführt. Die Membrananlage besteht aus Standard Platten Modulen der Fa. Dow (Typ DDS 30-4.5). Die Membranfläche der 1. Stufe beträgt 1,4 m² und die der 2. Stufe 0,2 m². Die Filtration wird bei einer Betriebstemperatur von 40°C durchgeführt. Innerhalb des Moduls wird das zu filtrierende Reaktionsgemisch jeweils längs der Membranoberfläche geführt. Die Überströmung beträgt dabei 0,5 - 2,5 m/sec. Das Reaktionsgemisch der Hydroformylierung wird vor der 1. Filtrationsstufe mit Toluol so weit verdünnt, daß der Toluol-Anteil bei 50 - 55 Gew.-% liegt. Anschließend wird das Reaktionsgemisch in der 1. Filtrationsstufe auf 50 %, bezogen auf die gesamte eingesetzte Menge, aufkonzentriert. Das nach der 1. Filtrationsstufe erhaltene Permeat wird nach Ergänzung geringer Mengen an Rhodium und DSA/TPPTS entsprechend den in Tabelle 2 aufgeführten Rh- bzw. P(III)-Verlusten der 2. Filtrationsstufe zugeführt. Die Retentate der beiden Stufen werden vereinigt und direkt wieder in die Hydroformylierungsreaktion zurückgeführt. Das Reaktionsprodukt der Hydroformylierung, der Tricyclodecandialdehyd, sowie auch gebildete Dicköle, permeieren neben dem Toluol unter dem herrschenden Arbeitsdruck von jeweils 1 MPa in der 1. und 2. Stufe die Membran und werden als Permeat der weiteren Aufarbeitung zugeführt. Hierzu wird zunächst das Toluol aus dem Permeat abdestilliert und in das Reaktionsgemisch der Hydroformylierung vor der Membranfiltration zurückgeführt, wobei das Reaktionsgemisch der Hydroformylierung auf 50 Gew.-% verdünnt wird.

Für die beiden Filtrationsstufen des Beispiels 7 werden die in Tabelle 3 dargestellten Flußleistungen ermittelt. Während der kontinuierlichen Versuchsdauer von über 12 Wochen ist kein Nachlassen der Aktivität festzustellen. Der größte Teil der P(III)-Verluste ist auf Phosphinoxid-Bildung zurückzuführen.

### Einfluß einer Druckkonditionierung der Polyaramidmembran auf die Trennleistung

In eine Laborzelle wird eine ungetemperte UF-PA 5 (PET 100)-Membran eingebaut und anschließend mit dem Reaktionsgemisch der Hydroformylierung aus Beispiel 7 beaufschlagt. Das unter den Membranfiltrationsbedingungen gemäß Beispiel 7 (siehe Tabelle 2) erhaltene Permeat und Retentat werden nach der Membranfiltration wieder vereinigt und in das Reaktionsgemisch der Hydroformylierung vor der Membranfiltration zurückgeführt. Die Rückhaltewerte für Phosphor(III) werden in Abhängigkeit von der Zeit analysiert (siehe Tabelle 3).

**Tabelle 3**

| | | | | | |
|---|---|---|---|---|---|
| Dauer der Druckkonditionierung [h] | 6 | 16 | 32 | 34 | 37 |
| P(III)-Rückhalt [% vom Einsatz] | 65 | 71 | >90 | >90 | >90 |

### Beispiele 8 - 11

Kontinuierliche Hydroformylierung von Dicyclopentadien unter Variation der Rhodium-Konzentration, bezogen auf DCP.

Die Hydroformylierung erfolgt kontinuierlich unter den in Tabelle 4 angegebenen Reaktionsbedingungen. Die anschließende Membranfiltration wird analog zu Beispiel 7 durchgeführt. Die erhaltenen Ergebnisse sind in Tabelle 4 zusammengefaßt. Zu erkennen ist, daß Aktivität und Selektivität mit zunehmender Rhodium-Konzentration steigen und sich die Membranfiltrationsergebnisse gleichzeitig verbessern.

### Beispiel 12 - 15:

Membranfiltration eines Reaktionsgemisches der kontinuierlichen Hydroformylierung von DCP unter Verwendung des Distearylammoniumsalzes des TPPTS als Ligand im Rh-Katalysatorsystem.

In den Beispielen 12 - 15 wird die Abhängigkeit zwischen der Toluol-Konzentration in dem zur Membranfiltration eingesetzten Reaktionsgemisch und der Flußleistung sowie den Rückhalteraten untersucht.

Zur Membranfiltration wird ein Reaktionsgemisch eingesetzt, das durch kontinuierliche Hydroformylierung unter den in Beispiel 6 angegebenen Bedingungen sowie einem Phosphor : Rhodium-Verhältnis von 76 erhalten wurde.

Die Membranfiltration wird einstufig bei einer Betriebstemperatur von 40°C, einem Druck von 1 MPa und einer Überströmung von 200 l/h über der Membran durchgeführt.

Die erhaltenen Flußleistungen sowie Rückhaltewerte sind in Tabelle 5 zusammengefaßt. Zu erkennen ist, daß sich ein zunehmender Toluol-Anteil in dem zur Membranfiltration eingesetzten Reaktionsgemisch sowohl günstig auf die Flußleistungen als auch auf die Rückhalteraten auswirkt.

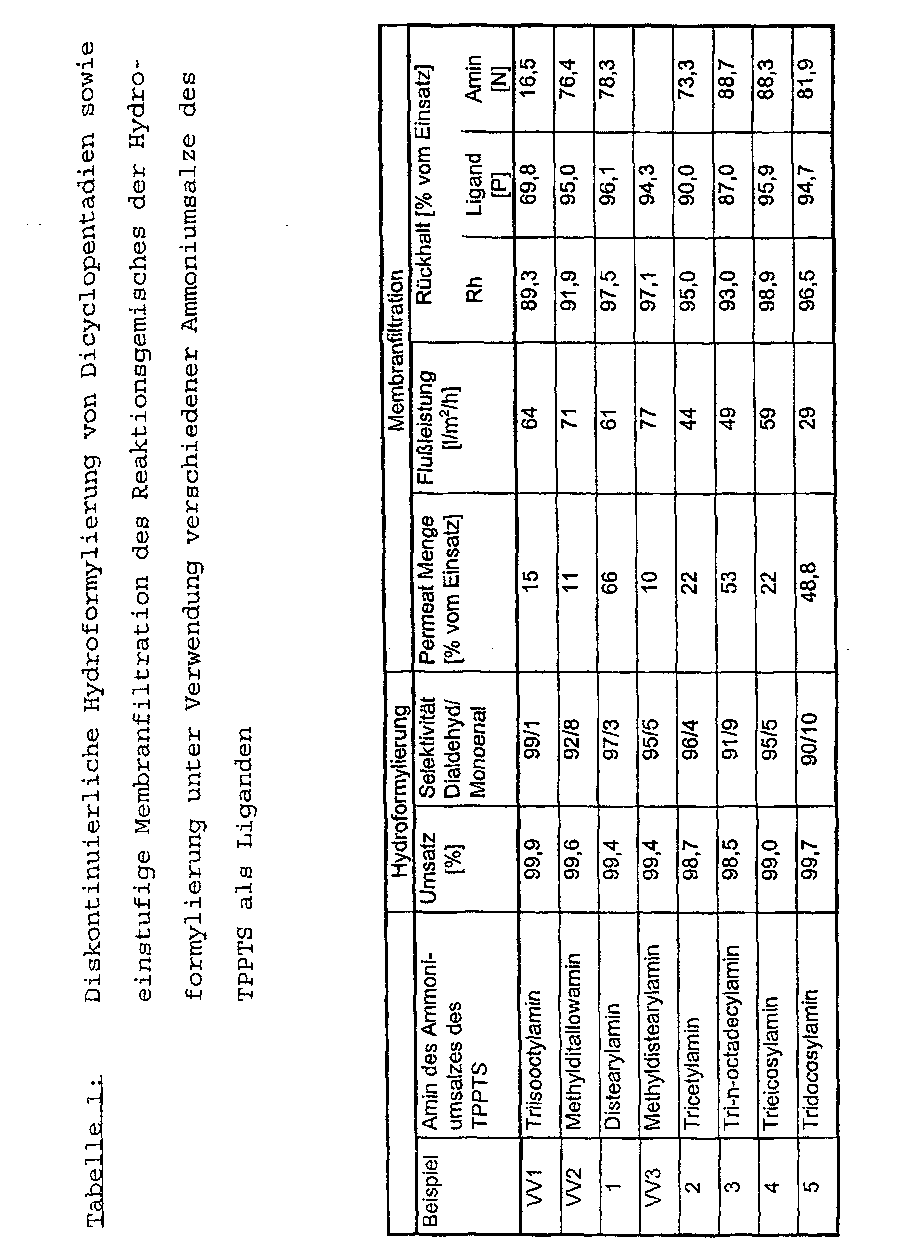

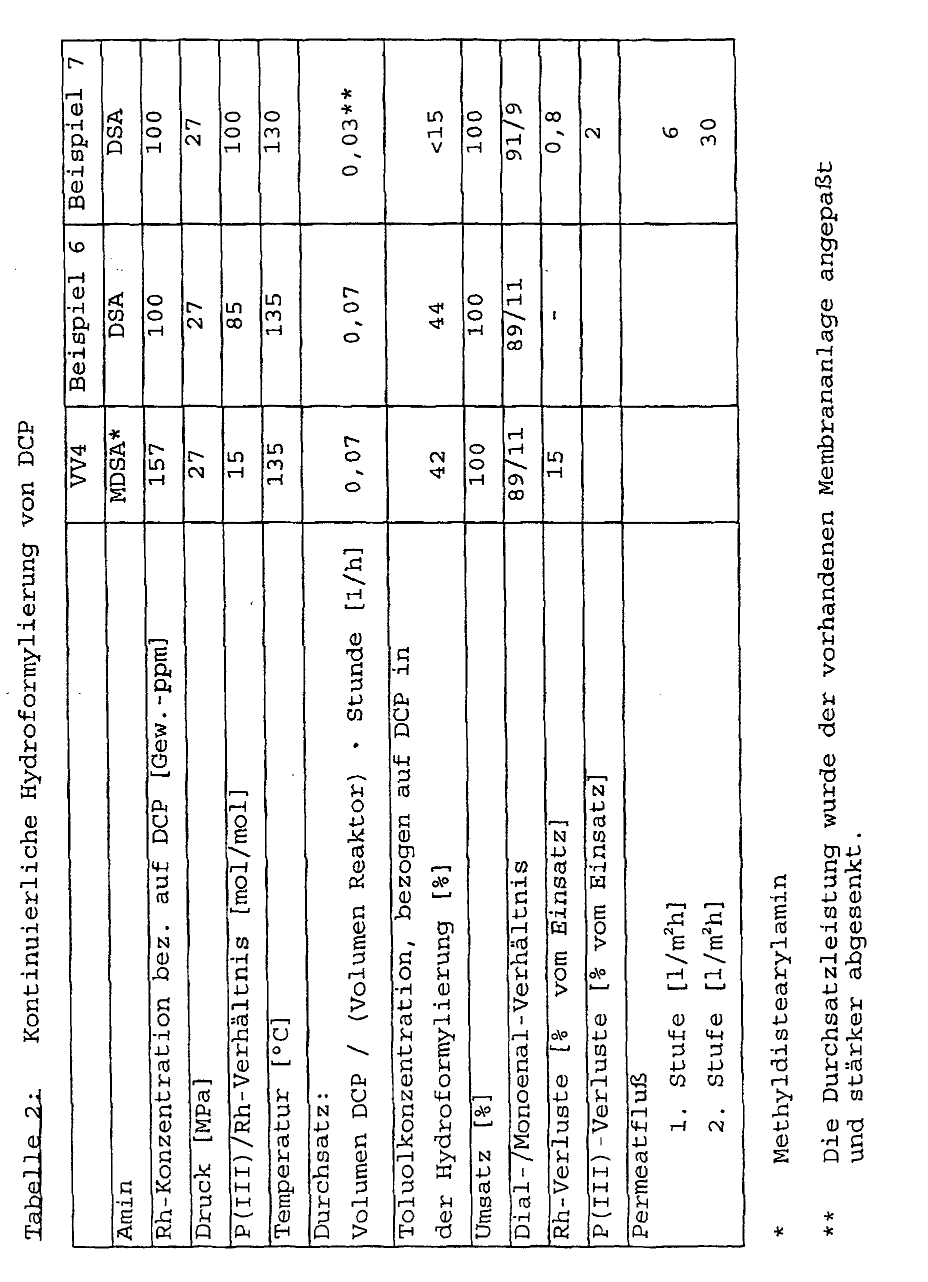

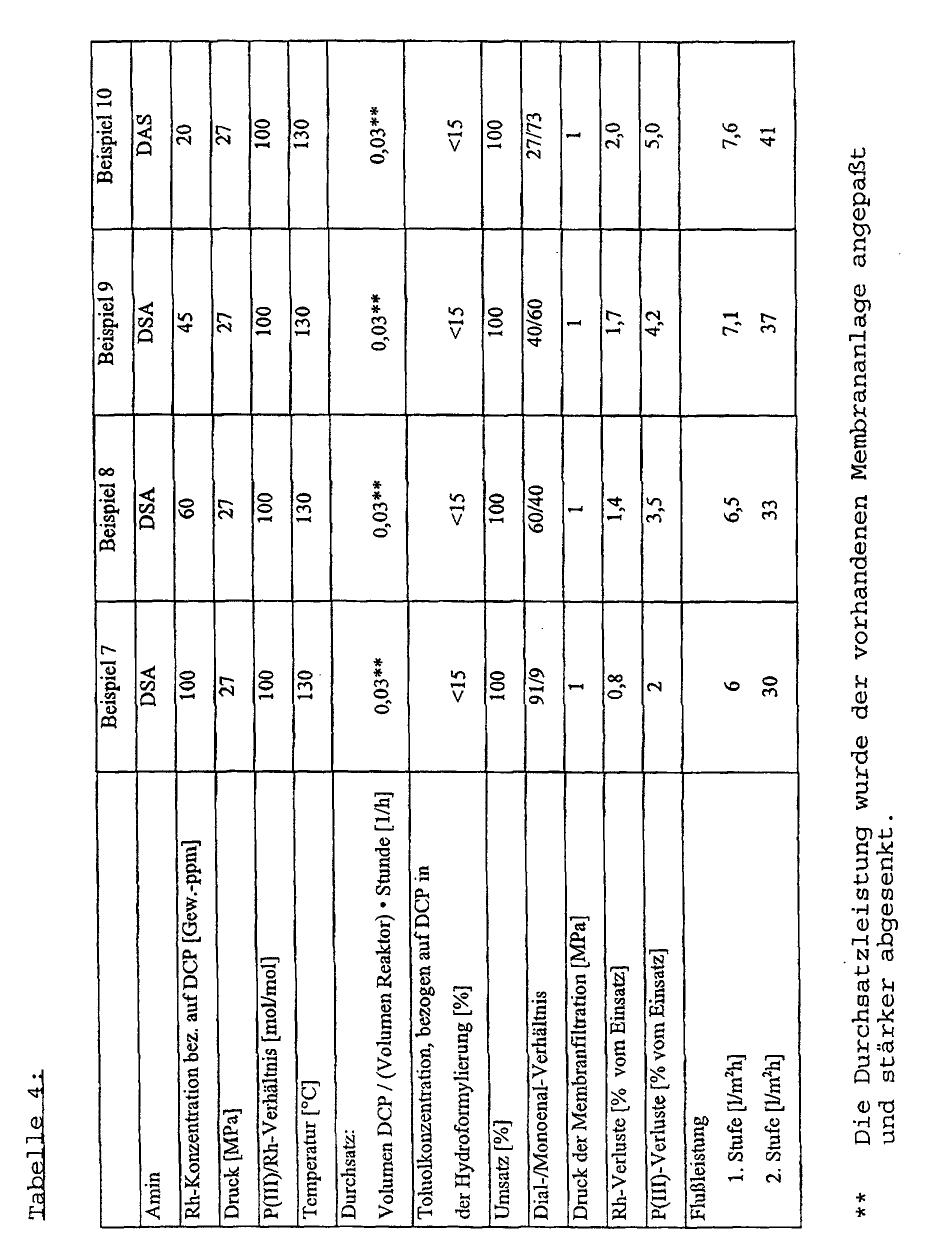

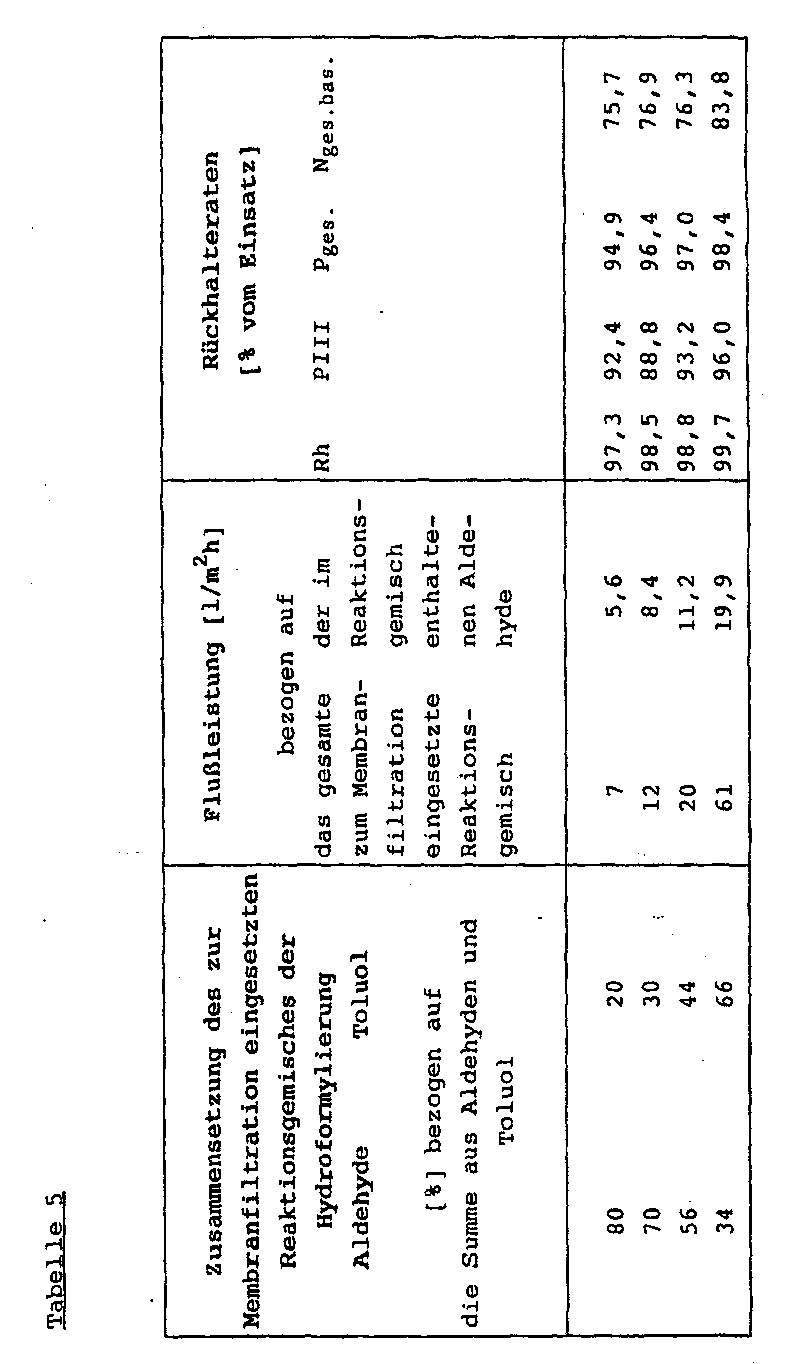

## Patentansprüche

1. Verfahren zur Herstellung von Aldehyden durch Hydroformylierung von olefinisch ungesättigten Verbindungen mit Wasserstoff und Kohlenmonoxid in homogener Phase in Gegenwart eines Rhodium-Komplexverbindungen sowie aromatische Phosphine im molaren Überschuß enthaltenden Katalysatorsystems und Abtrennung des Katalysatorsystems vom Reaktionsgemisch der Hydroformylierung durch Druckfiltration an einer semipermeablen Membran aus einem aromatischen Polyamid, **dadurch gekennzeichnet, daß** die Hydroformylierung bei einem pH-Wert von 2,5 bis 4,3 unter Verwendung eines molaren Phosphin : Rhodium-Verhältnisses von mindestens 60 und bei einer Rhodium-Konzentration von mindestens 10 Gew.-ppm, bezogen auf die eingesetzte olefinisch ungesättigte Verbindung, durchgeführt wird und als aromatische Phosphine Alkyl- und/oder Arylammoniumsalze sulfonierter oder carboxylierter Triarylphosphine der allgemeinen Formel I 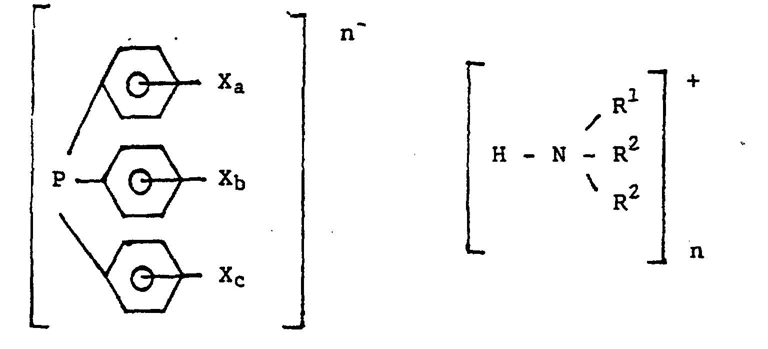 eingesetzt werden, worin X einen Sulfonat-(SO₃⁻)-oder Carboxylat-(COO⁻)-Rest bedeutet, a, b und c gleich oder verschieden und 0 oder 1 sind, wobei mindestens einer der Parameter a, b oder c gleich 1 sein muß, n gleich 1, 2 oder 3 ist, R¹ und R² gleich oder verschieden und C₈ - C₃₀- Alkylreste oder C₆ - C₁₀-Aryl- oder -Cycloalkylreste bedeuten und R¹ auch Wasserstoff sein kann und die Summe der Kohlenstoffatome in den Resten R¹ und R² mindestens 30 betragen muß.

2. Verfahren nach Anspruch 1, **dadurch gekennzeichnet, daß** X in der allgemeinen Formel I für einen Sulfonatrest steht.

3. Verfahren nach Anspruch 1 oder 2, **dadurch gekennzeichnet, daß** R¹ und R² in der allgemeinen Formel I gleich oder verschieden sind und für C₁₂ - C₂₂-Alkyl- oder einen Phenyl- oder Cyclohexylrest stehen.

4. Verfahren nach einem oder mehreren der Ansprüche 1 - 3, **dadurch gekennzeichnet, daß** a, b und c gleich 1 sind.

5. Verfahren nach einem oder mehreren der Ansprüche 1 - 4, **dadurch gekennzeichnet, daß** die Ammonium-Kationen [HNR¹R²R²]⁺ in der allgemeinen Formel I insgesamt mindestens 30 und maximal 90, bevorzugt 32- 70 und insbesondere 36 - 54 Kohlenstoffatome in den Resten R¹ und R² enthalten.

6. Verfahren nach Anspruch 5, **dadurch gekennzeichnet, daß** es sich bei den Ammonium-Kationen [HNR¹R²R²]⁺ in der allgemeinen Formel I um das Distearylammoniumion, das Tricetylammoniumion oder das Tri-n-octadecylammoniumion handelt.

7. Verfahren nach einem oder mehreren der Ansprüche 1 - 6, **dadurch gekennzeichnet, daß** das molare Verhältnis von Rhodium zu aromatischem Phosphin der allgemeinen Formel I 1 : (60-120), bevorzugt 1 : (70-110), insbesondere 1 : (80-100) beträgt.

8. Verfahren nach einem oder mehreren der Ansprüche 1-7, **dadurch gekennzeichnet, daß** die Rh-Konzentration in der Hydroformylierung, bezogen auf die olefinisch ungesättigte Verbindung, mindestens 20 Gew.-ppm, bevorzugt 60-150 Gew.-ppm, beträgt.

9. Verfahren nach einem oder mehreren der Ansprüche 1-8, **dadurch gekennzeichnet, daß** das Katalysatorsystem in einem dem Verfahren vorgeschalteten Schritt hergestellt wird oder in-situ während des Verfahrens gebildet wird.

10. Verfahren nach Anspruch 9, **dadurch gekennzeichnet, daß** zur Herstellung des Katalysatorsystems in einem vorgeschalteten Schritt die Rhodium-Komponente und das Triarylphosphin der Formel I, jeweils in einem organischen Lösungsmittel gelöst oder suspendiert, zusammengebracht werden und unter einem Kohlenmonoxid/Wasserstoff-Druck von 15-25 MPa bei einer Temperatur von 80-150°C mindestens 1 Stunde umgesetzt werden.

11. Verfahren nach einem oder mehreren der Ansprüche 1 bis 10, **dadurch gekennzeichnet, daß** als olefinisch ungesättigte Verbindungen substituierte ode unsubstituierte Alkene mit 2 bis 30 Kohlenstoffatomen, substituierte oder unsubstituierte Diene mit 4 bis 10 Kohlenstoffatomen, substituierte oder unsubstituierte Cycloalkene oder Dicycloalkene mit 5 bis 12 Kohlenstoffatomen im Ringsystem, Ester einer ungesättigten Carbonsäure mit 3 bis 20 Kohlenstoffatomen und eines aliphatischen Alkohols mit 1 bis 18 Kohlenstoffatomen, Ester einer gesättigten Carbonsäure mit 2 bis 20 Kohlenstoffatomen und eines ungesättigten Alkohols mit 2 bis 18 Kohlenstoffatomen, ungesättigte Alkohole oder Ether mit jeweils 3 bis 20 Kohlenstoffatomen oder araliphatische Olefine mit 8 bis 20 Kohlenstoffatomen verwendet werden.

12. Verfahren nach Anspruch 11, **dadurch gekennzeichnet, daß** als olefinisch ungesättigte Verbindung Dicyclopentadien eingesetzt wird.

13. Verfahren nach einem oder mehreren der Ansprüche 1 - 12, **dadurch gekennzeichnet, daß** die Hydroformylierung bei einer Temperatur von 100 - 140°C, bevorzugt 120 - 130°C und unter einem Druck von 0,5 - 27 MPa, bevorzugt 20 - 25 MPa, durchgeführt wird.

14. Verfahren nach einem oder mehreren der Ansprüche 1 - 13, **dadurch gekennzeichnet, daß** die Hydroformylierung bei einem pH-Wert von 3,0 - 4,0, insbesondere 3,5, durchgeführt wird.

15. Verfahren nach einem oder mehreren der Ansprüche 1 - 14, **dadurch gekennzeichnet, daß** in Anwesenheit eines organischen Lösungsmittels gearbeitet wird.

16. Verfahren nach Anspruch 15, **dadurch gekennzeichnet, daß** die Lösungsmittelkonzentration in der Hydroformylierungsreaktion 5 - 25 Gew.-%, bevorzugt 7 - 13 Gew.-%, bezogen auf das gesamte Reaktionsgemisch der Hydroformylierung, und in der Membranfiltration 30 - 70 Gew.-%, bevorzugt 40 - 60 Gew.-%, bezogen auf das gesamte zur Membranfiltration eingesetzte Reaktionsgemisch, beträgt.

17. Verfahren nach einem oder mehreren der Ansprüche 1 - 16, **dadurch gekennzeichnet, daß** in dem zur Membranfiltration eingesetzten Reaktionsgemisch der Hydroformylierung die Konzentration der im Überschuß vorliegenden aromatischen Phosphine der allgemeinen Formel I 2,5 - 25 Gew.-%, bevorzugt 5 - 15 Gew.-%, bezogen auf das gesamte zur Membranfiltration eingesetzte Reaktionsgemisch, beträgt.

18. Verfahren nach einem oder mehreren der Ansprüche 1 - 17, **dadurch gekennzeichnet, daß** die Konzentration der Rhodium-Komplexverbindungen in dem zur Membranfiltration eingesetzten Reaktionsgemisch der Hydroformylierung 2 - 400 Gew.-ppm, bevorzugt 10 - 300 Gew.-ppm, insbesondere 50 - 150 Gew.-ppm, bezogen auf das gesamte zur Membranfiltration eingesetzte Reaktionsgemisch, beträgt.

19. Verfahren nach einem oder mehreren der Ansprüche 1 - 18, **dadurch gekennzeichnet, daß** die Membranfiltration unter einem Druck von 0,1 - 15, bevorzugt 0,5 - 5 und insbesondere 1 - 2 MPa erfolgt und ein- oder mehrstufig, bevorzugt zweistufig, durchgeführt wird.

20. Verfahren nach einem oder mehreren der Ansprüche 1 - 19, **dadurch gekennzeichnet, daß** die Membranfiltration mit in Reihe geschalteten Trennstufen durchgeführt wird.

21. Verfahren nach einem oder mehreren der Ansprüche 1 - 20, **dadurch gekennzeichnet, daß** die Gesamtretentatmenge der Membranfiltration 8 - 90, bevorzugt 10 - 70, besonders bevorzugt 15 - 50 und insbesondere 20 - 40 %, bezogen auf das zur Membranfiltration eingesetzte Reaktionsgemisch, beträgt und die Konzentration der abgetrennten aromatischen Phosphine der allgemeinen Formel I im Retentat mindestens dreimal so groß ist wie in dem zur Membranfiltration eingesetzten Reaktionsgemisch der Hydroformylierung.

22. Verfahren nach einem oder mehreren der Ansprüche 1 - 21, **dadurch gekennzeichnet, daß** bei der zweistufigen Membranfiltration das Verhältnis der Retentatmenge der 1. Filtrationsstufe zur Retentatmenge der 2. Stufe etwa 1 : 1 beträgt.

23. Verfahren nach einem oder mehreren der Ansprüche 1 - 22, **dadurch gekennzeichnet, daß** die das Katalysatorsystem enthaltenden Retentate der Trennstufen der Membranfiltration wieder in die Hydroformylierung zurückgeführt werden, gegebenenfalls unter ergänzendem Zusatz des Rhodium und/oder der Rhodium-Komplexverbindungen sowie der aromatischen Phosphine der allgemeinen Formel I.

24. Verfahren nach Anspruch 23, **dadurch gekennzeichnet, daß** bei zweistufiger Durchführung der Membranfiltration der ergänzende Zusatz des Rhodiums und/oder der Rhodium-Komplexverbindungen sowie der aromatischen Phosphine der allgemeinen Formel I bereits zum Permeat der 1. Filtrationsstufe vor dessen Zuleitung zur 2. Filtrationsstufe erfolgt.

25. Verfahren nach einem oder mehreren der Ansprüche 1 - 24, **dadurch gekennzeichnet, daß** aus den vereinigten Permeaten der Trennstufen der Membranfiltration das Lösungsmittel destillativ abgetrennt wird, vor die Membranfiltration zurückgeführt und dem Reaktionsgemisch der Hydroformylierung vor der Membranfiltration zugesetzt wird.

## Claims

1. A process for preparing aldehydes by hydroformylation of olefinically unsaturated compounds with hydrogen and carbon monoxide in homogeneous phase in the presence of a catalyst system comprising rhodium complex compounds and aromatic phosphines in a molar excess and separating off the catalyst system from the hydroformylation reaction mixture by pressure filtration on a semipermeable membrane of an aromatic polyamide, which comprises carrying out the hydroformylation at a pH of 2.5 to 4.3 using a molar ratio of phosphine:rhodium of at least 60 and at a rhodium concentration of at least 10 ppm by weight, based on the olefinically unsaturated compound used, and using, as aromatic phosphines, alkylammonium and/or arylammonium salts of sulfonated or carboxylated triarylphosphines of the formula I 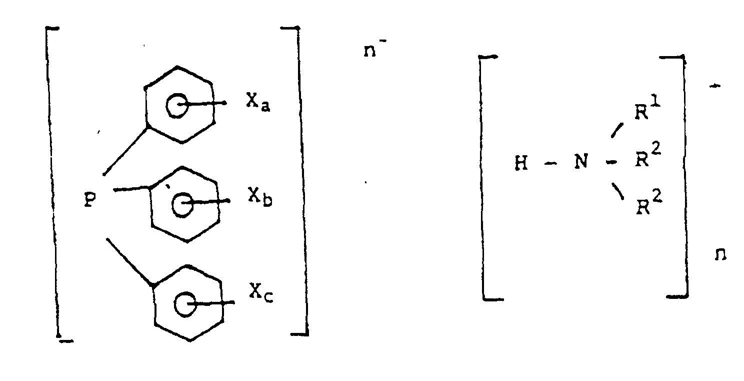 in which X is a sulfonate (SO₃⁻) or carboxylate (COO⁻) radical, a, b and c are identical or different and are 0 or 1, where at least one of the parameters a, b or c must be equal to 1, n is equal to 1, 2 or 3, R¹ and R² are identical or different and are C₈-C₃₀-alkyl radicals or C₆-C₁₀-aryl radicals or C₆-C₁₀-cycloalkyl radicals, and R¹ can also be hydrogen, and the total of the carbon atoms in the radicals R¹ and R² must be at least 30.

2. The process as claimed in claim 1, wherein X in the formula I is a sulfonate radical.

3. The process as claimed in claim 1 or 2, wherein R¹ and R² in the formula I are identical or different and are C₁₂-C₂₂-alkyl or a phenyl or cyclohexyl radical.

4. The process as claimed in one or more of claims 1-3, wherein a, b and c are equal to 1.

5. The process as claimed in one or more of claims 1-4, wherein the ammonium cations [HNR¹R²R²]⁺ in the formula I contain in total at least 30 and at most 90, preferably 32-70, and in particular 36-54, carbon atoms in the radicals R¹ and R².

6. The process as claimed in claim 5, wherein the ammonium cations [HNR¹R²R²]⁺ in the formula I are the distearylammonium ion, the tricetylammonium ion or the tri-n-octadecylammonium ion.

7. The process as claimed in one or more of claims 1-6, wherein the molar ratio of rhodium to aromatic phosphine of the formula I is 1:(60-120), preferably 1: (70-110)/ in particular 1: (80-100).

8. The process as claimed in one or more of claims 1-7, wherein the Rh concentration in the hydroformylation, based on the olefinically unsaturated compound, is at least 20 ppm by weight, preferably 60-150 ppm by weight.

9. The process as claimed in one or more of claims 1-8, wherein the catalyst system is prepared in a step upstream of the process or is formed in situ during the process.

10. The process as claimed in claim 9, wherein, to prepare the catalyst system in an upstream step, the rhodium component and the triaryl phosphine of the formula I, each dissolved or suspended in an organic solvent, are brought together and reacted for at least 1 hour at a temperature of 80-150°C under a carbon monoxide/hydrogen pressure of 15-25 MPa.

11. The process as claimed in one or more of claims 1 to 10, wherein the olefinically unsaturated compounds used are substituted or unsubstituted alkenes having 2 to 30 carbon atoms, substituted or unsubstituted dienes having 4 to 10 carbon atoms, substituted or unsubstituted cycloalkenes or dicycloalkenes having 5 to 12 carbon atoms in the ring system, esters of an unsaturated carboxylic acid having 3 to 20 carbon atoms and of an aliphatic alcohol having 1 to 18 carbon atoms, esters of a saturated carboxylic acid having 2 to 20 carbon atoms and of an unsaturated alcohol having 2 to 18 carbon atoms, unsaturated alcohols or ethers each having 3 to 20 carbon atoms or araliphatic olefins having 8 to 20 carbon atoms.

12. The process as claimed in claim 11, wherein the olefinically unsaturated compound used is dicyclopentadiene.

13. The process as claimed in one or more of claims 1-12, wherein the hydroformylation is carried out at a temperature of 100-140°C, preferably 120-130°C and at a pressure of 0.5-27 MPa, preferably 20-25 MPa.

14. The process as claimed in one or more of claims 1-13, wherein the hydroformylation is carried out at a pH of 3.0-4.0, in particular 3.5.

15. The process as claimed in one or more of claims 1-14, wherein it is carried out in the presence of an organic solvent.

16. The process as claimed in claim 15, wherein the solvent concentration in the hydroformylation reaction is 5-25% by weight, preferably 7-13% by weight, based on the total hydroformylation reaction mixture, and in the membrane filtration is 30-70% by weight, preferably 40-60% by weight, based on the total reaction mixture used for the membrane filtration.

17. The process as claimed in one or more of claims 1-16, wherein, in the hydroformylation reaction mixture used for the membrane filtration, the concentration of the aromatic phosphines of the formula I present in excess is 2.5-25% by weight, preferably 5-15% by weight, based on the total reaction mixture used for the membrane filtration.

18. The process as claimed in one or more of claims 1-17, wherein the concentration of the rhodium complex compounds in the hydroformylation reaction mixture used for the membrane filtration is 2-400 ppm by weight, preferably 10-300 ppm by weight, in particular 50-150 ppm by weight, based on the total reaction mixture used for the membrane filtration.

19. The process as claimed in one or more of claims 1-18, wherein the membrane filtration is performed at a pressure of 0.1-15, preferably 0.5-5, and in particular 1-2, MPa and is carried out as a single-stage or multistage process, preferably as a two-stage process.

20. The process as claimed in one or more of claims 1-19, wherein the membrane filtration is carried out with series-connected separation stages.

21. The process as claimed in one or more of claims 1-20, wherein the total amount of retentate of the membrane filtration is 8-90, preferably 10-70, particularly preferably 15-50, and, in particular, 20-40, %, based on the reaction mixture used for the membrane filtration, and the concentration of the separated aromatic phosphines of the formula I in the retentate is at least three times as high as that in the hydroformylation mixture used for the membrane filtration.

22. The process as claimed in one or more of claims 1-21, wherein, in the two-stage membrane filtration, the ratio of the amount of retentate of the 1st filtration stage to the amount of retentate of the 2nd stage is about 1:1.

23. The process as claimed in one or more of claims 1-22, wherein the retentates of the membrane filtration separation stages which contain the catalyst system are recycled to the hydroformylation, if appropriate with supplementary addition of the rhodium and/or the rhodium complex compounds and of the aromatic phosphines of the formula I.

24. The process as claimed in claim 23, wherein, in the case of the two-stage membrane filtration procedure, the supplementary addition of the rhodium and/or of the rhodium complex compounds and of the aromatic phosphines of the formula I is made in advance to the permeate of the 1st filtration stage prior to its feed to the 2nd filtration stage.

25. The process as claimed in one or more of claims 1-24, wherein the solvent is separated off by distillation from the combined permeates of the membrane separation stages, recycled upstream of the membrane filtration and added to the hydroformylation reaction mixture upstream of the membrane filtration.

## Revendications

1. Procédé de préparation d'aldéhydes par hydroformylation de composés insaturés oléfiniquement avec de l'hydrogène et du monoxyde de carbone en phase homogène en présence d'un système catalytique contenant des composés complexes de rhodium ainsi que de phosphines aromatiques en excès moléculaire et séparation du système catalytique du mélange réactionnel d'hydroformylation par filtration sous pression sur une membrane semi-perméable en polyamide aromatique, **caractérisé en ce qu'**on effectue l'hydroformylation à une valeur de pH de 2,5 à 4,3 en utilisant un rapport molaire phosphine : rhodium d'au moins 60 et à une concentration de rhodium d'au moins 10 ppm en poids, rapporté au composé insaturé oléfiniquement utilisé et **en ce qu'**on utilise comme phosphines aromatiques des sels d'alkylammonium et/ou d'arylammonium de triarylphosphines sulfonées ou carboxylées de formule générale I 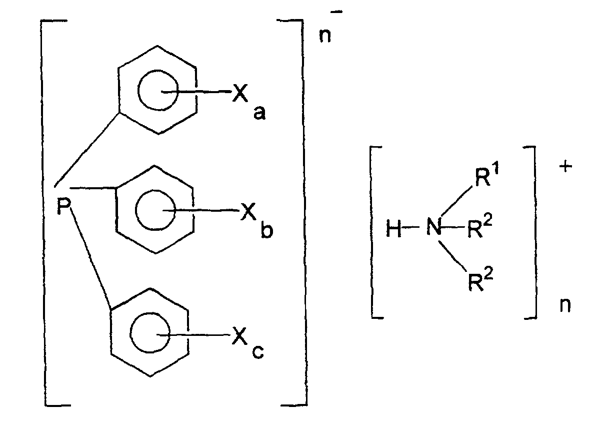 dans laquelle X est un radical sulfonate -(SO₃⁻)- ou un radical carboxylate -(COO⁻)-, a b et c sont identiques ou différents et valent 0 ou 1, au moins un des paramètres a, b ou c devant être égal à 1, n est égal à 1, 2 ou 3, R¹ et R² sont identiques ou différents et sont un radical alkyle en C₈-C₃₀ ou un radical aryle ou cycloalkyle en C₆-C₁₀ et R¹ peut en outre également être l'hydrogène et la somme des atomes de carbone dans les radicaux R¹ et R² doit être au moins 30.

2. Procédé selon la revendication 1, **caractérisé en ce que** X dans la formule générale I est un radical sulfonate.

3. Procédé selon la revendication 1 ou 2, **caractérisé en ce que** R¹ et R² dans la formule générale I sont identiques ou différents et sont un radical alkyle en C₁₂-C₂₂ ou un radical phényle ou un radical cyclohexyle.

4. Procédé selon l'une ou plusieurs des revendications 1 à 3, **caractérisé en ce que** a, b et c sont égaux à 1.

5. Procédé selon l'une ou plusieurs des revendications 1 à 4, **caractérisé en ce que** les cations ammonium [HNR¹R²R²]⁺ dans la formule générale I contiennent au total au moins 30 et au plus 90, de préférence 32 - 70 et en particulier 36 - 54 atomes de carbone dans les radicaux R¹ et R².

6. Procédé selon la revendication 5, **caractérisé en ce que** les cations ammonium [HNR¹R²R²]⁺ dans la formule générale I sont l'ion distéarylammonium, l'ion tricétylammonium ou l'ion tri-n-octadécylammonium.

7. Procédé selon l'une ou plusieurs des revendications 1 à 6, **caractérisé en ce que** le rapport molaire du rhodium à la phosphine aromatique de formule générale I est 1 : (60 - 120), de préférence 1 : (70 - 110), en particulier 1 : (80-100).

8. Procédé selon l'une ou plusieurs des revendications 1 à 7, **caractérisé en ce que** la concentration de Rh dans l'hydroformylation, rapporté au composé insaturé oléfiniquement, est au moins 20 ppm en poids, de préférence 60 - 150 ppm en poids.

9. Procédé selon l'une ou plusieurs des revendications 1 à 8, **caractérisé en ce que** le système catalytique est préparé dans une étape en amont du procédé ou est formé *in situ* pendant le procédé.

10. Procédé selon la revendication 9, **caractérisé en ce que** pour préparer le système catalytique dans une étape en amont, le composant rhodium et la triarylphosphine de formule I, chacun dissous ou en suspension dans un solvant organique, sont mis ensemble et mis à réagir sous une pression de monoxyde de carbone/hydrogène de 15 - 25 MPa à une température de 80 - 150°C pendant au moins 1 heure.

11. Procédé selon l'une ou plusieurs des revendications 1 à 10, **caractérisé en ce qu'**on utilise comme composés insaturés oléfiniquement des alcènes substitués ou non substitués ayant 2 à 30 atomes de carbone, des diènes substitués ou non substitués ayant 4 à 10 atomes de carbone, des cycloalcènes ou des dicycloalcènes substitués ou non substitués ayant 5 à 12 atomes de carbone dans le système cyclique, des esters d'un acide carboxylique insaturé ayant 3 à 20 atomes de carbone et d'un alcool aliphatique ayant 1 à 18 atomes de carbone, des esters d'un acide carboxylique insaturé ayant 2 à 20 atomes de carbone et d'un alcool insaturé ayant 2 à 18 atomes de carbone, des alcools ou des éthers insaturés ayant chacun 3 à 20 atomes de carbone ou des oléfines araliphatiques ayant 8 à 20 atomes de carbone.

12. Procédé selon la revendication 11, **caractérisé en ce qu'**on utilise comme composé insaturé oléfiniquement le dicyclopendadiène.

13. Procédé selon l'une ou plusieurs des revendications 1 à 12, **caractérisé en ce qu'**on effectue l'hydroformylation à une température de 100 - 140°C, de préférence 120 - 130°C et sous une pression de 0,5 - 27 MPa, de préférence 20 - 25 MPa.

14. Procédé selon l'une ou plusieurs des revendications 1 à 13, **caractérisé en ce qu'**on effectue l'hydroformylation à une valeur de pH de 3,0- 4,0, en particulier 3,5.

15. Procédé selon l'une ou plusieurs des revendications 1 à 14, **caractérisé en ce qu'**on travaille en présence d'un solvant organique.

16. Procédé selon la revendication 15, **caractérisé en ce que** la concentration du solvant dans l'hydroformylation est 5 - 25% en poids, de préférence 7 - 13% en poids, rapporté au mélange réactionnel total d'hydroformylation, et dans la filtration par membrane est 30 - 70% en poids, de préférence 40 - 60% en poids, rapporté au mélange réactionnel total utilisé pour la filtration par membrane.

17. Procédé selon l'une ou plusieurs des revendications 1 à 16, caractérisé en ce le mélange réactionnel d'hydroformylation utilisé pour la filtration par membrane, la concentration des phosphines aromatiques de formule générale I présentes en excès est 2,5 - 25% en poids, de préférence 5 - 15% en poids, rapporté au mélange réactionnel total utilisé pour la filtration par membrane.

18. Procédé selon l'une ou plusieurs des revendications 1 à 17, caractérisé en ce la concentration des composés complexes de rhodium dans le mélange réactionnef d'hydroformylation utilisé pour la filtration par membrane est 2 - 400 ppm en poids, de préférence 10 - 300 ppm en poids, en particulier 50 - 150 ppm en poids, rapporté au mélange réactionnel total utilisé pour la filtration par membrane.

19. Procédé selon l'une ou plusieurs des revendications 1 à 18,
**caractérisé en ce que** la filtration par membrane est effectuée à une pression de 0,1 - 15, de préférence 0,5 - 5 et en particulier 1 - 2 MPa et est effectuée comme un procédé en une étape ou en étapes multiples, de préférence en deux étapes.

20. Procédé selon l'une ou plusieurs des revendications 1 à 19, **caractérisé en ce que** la filtration par membrane est effectuée avec des étapes de séparation reliées en série.

21. Procédé selon l'une ou plusieurs des revendications 1 à 20, **caractérisé en ce que** la quantité de rétentatsde filtration par membrane est 8 - 90, de préférence 10 - 70, mieux encore 15 - 50 et en particulier 20 - 40%, rapporté au mélange réactionnel utilisé pour la filtration par membrane, et la concentration des phosphines aromatiques séparées de formule I dans le rétentat est au moins trois fois aussi élevée que celle dans le mélange réactionnel d'hydroformylation utilisé pour la filtration par membrane.

22. Procédé selon l'une ou plusieurs des revendications 1 à 21, **caractérisé en ce que** dans la filtration par membrane en deux étapes, le rapport de la quantité de rétentat de la 1^{ère} étape filtration à la quantité de rétentat de la 2^{ème} étape est environ 1 : 1.

23. Procédé selon l'une ou plusieurs des revendications 1 à 22, **caractérisé en ce que** les rétentats des étapes de séparation de filtration par membrane qui contiennent le système catalytique sont recyclés à l'hydroformylation, en option avec une addition supplémentaire de rhodium et/ou de composés complexes de rhodium et de phosphines aromatiques de formule I.

24. Procédé selon la revendication 23, **caractérisé en ce que** dans le cas d'un mode opératoire de filtration par membrane en deux étapes, l'addition supplémentaire de rhodium et/ou des composés complexes de rhodium et de phosphines aromatiques de formule générale I est faite au préalable au perméat de la 1^{ère} étape de filtration avant son introduction dans la 2^{ème} étape de filtration.

25. Procédé selon l'une ou plusieurs des revendications 1 à 24, **caractérisé en ce que** le solvant est séparé par distillation des perméats combinés des étapes de séparation par membrane, recyclé en amont de la filtration par membrane et ajouté au mélange réactionnel d'hydroformylation en amont de la filtration par membrane.
